# EUROPÄISCHE PATENTSCHRIFT

(11) **EP 2 160 168 B1**
(45) Veröffentlichungstag und Bekanntmachung des Hinweises auf die Patenterteilung: **07.12.2011**
(21) Anmeldenummer: 08773588.2
(22) Anmeldetag: 23.06.2008
(51) Int. Cl.: A61H 1/00, A63B 22/16, A63B 24/00, A63B 21/072

(54) **VORRICHTUNG UND VERFAHREN FÜR EIN TRAINING UND/ODER EINE ANALYSE DES BEWEGUNGSAPPARATS EINES BENUTZERS**
APPARATUS AND METHOD FOR EXERCISE AND/OR ANALYSIS OF THE LOCOMOTOR SYSTEM OF A USER
PROCÉDÉ ET DISPOSITIF D'ENTRAÎNEMENT ET/OU D'ANALYSE DE L'APPAREIL MOTEUR D'UN UTILISATEUR

(30) Priorität: 22.06.2007 DE 102007029311; 22.06.2007 DE 202007008887 U
(43) Veröffentlichungstag der Anmeldung: 10.03.2010
(73) Patentinhaber: Schiessl, Hans, 75177 Pforzheim (DE)
(72) Erfinder: Schiessl, Hans, 75177 Pforzheim (DE)
(74) Vertreter: Leitner, Waldemar
(86) Internationale Anmeldenummer: PCT/EP2008/005051
(87) Internationale Veröffentlichungsnummer: WO 2009/000487

(56) Entgegenhaltungen:
- WO-A-2006/070429
- DE-A1-102007 014 080
- DE-T2- 60 111 016
- US-A1- 2001 000 782
- US-A1- 2006 229 159

## Beschreibung

Die vorliegende Erfindung betrifft eine Vorrichtung für Training und/oder eine Analyse eines Bewegungsapparats eines Benutzers, wobei die Vorrichtung mindestens ein Grundelement, das durch eine Antriebseinrichtung in periodische und/oder aperiodische Bewegungen zur Erzeugung einer auf den Benutzer einwirkenden Anregungsfunktion versetzbar ist, und mindestens einen Sensor zur Ermittlung einer Antwortfunktion eines Benutzers der Vorrichtung auf die durch das Grundelement auf ihn aufgeprägte Anregungsfunktion, dessen oder deren Sensorsignale einer Auswerteeinrichtung der Vorrichtung zugeführt sind, aufweist.

Eine derartige Vorrichtung sowie ein derartiges Verfahren sind bekannt. Zum Beispiel ist aus der EP 0 929 284 der Anmelderin eine Vorrichtung bekannt, bei der über eine Grundplatte der Vorrichtung eine periodische, sinusförmige Bewegung seitenalternierend auf die Fußsohlen des Benutzers aufgebracht wird, wobei die vorzugsweise in einem Zustand mit erhöhtem Grundtonus befindlichen Muskeln durch eine Gegenbewegung reagieren und dadurch trainiert werden.

Weiterhin ist es bekannt, die zwischen den Fußsohlen des Benutzers und der Grundplatte der Vorrichtung wirkenden Drücke mittels Sensoren zu ermitteln.

Die bekannten Vorrichtungen und Verfahren Weisen aber den Nachteil auf, dass keine Korrelation zwischen der von der Vorrichtung hervorgerufenen Anregung und der Antwort des Benutzers darauf ermittelt werden kann.

Die DE 198 46 982 beschreibt ein Verfahren und ein System zur Überwachung der Haltung eines Benutzers an einem Trainingsgerät, wobei am Körper des Benutzers und/oder an bestimmten Stellen des Trainingsgeräts Sensoren angebracht werden. Die von den Sensoren erfassten Messdaten werden in einer Auswerteeinrichtung analysiert, um eine fehlerhafte Bewegung oder Haltung des Benutzers während der Benutzung des Trainingsgeräts zu erkennen und in diesem Fall dem Benutzer oder dem Trainingsgerät eine entsprechende Rückmeldung auszugeben. Das aus der Druckschrift bekannte Verfahren und das System zur Überwachung der Haltung eines Benutzers an einem Trainingsgerät ist auf die Erfassung der Bewegung und Haltung des Benutzers und/oder von ihm beaufschlagter Bauteile des Trainingsgeräts beschränkt und das Trainingsgerät selbst erlaubt keine aktive Anregung des Bewegungsapparat des Benutzers derart, dass eine Anregungsfunktion aufgeprägt wird.

Die DE 20 2005 002 086 beschreibt eine Vibrationstrainingsgerät, welches ein Gehäuse mit einer Schwingungsplattform und einen Schwingungsgenerator aufweist, der mit der Schwingungsplattform verbunden ist, wobei ein Steuergerät den Schwingungsgenerator regelt. Eine Schwingungssensoreinheit ist am Körper des Benutzers befestigbar und die am Körper aufgenommenen Schwingungen werden in Form von Signalen an das Steuergerät übertragen. Dieses regelt dann den Schwingungsgenerator in Anpassung an die Körperschwingungen. Indem durch diesen Regelkreis die aufgenommenen Körperschwingungen mit voreingestellten Grenzwerten kontinuierlich verglichen werden und eine Regelung durch das Steuergerät vorliegt, wird erreicht, dass die über die Schwingungsplattform in den Körper eingeleiteten Vibrationen keine schädliche Wirkung entfalten, da beim 0-berschreiten dieser Grenzwerte das Steuergerät mittels einer Abschaltautomatik den Schwingungsgenerator heruntersetzt oder ihn sogar stillsetzt.

Aus der DE 196 34 396 ist ein Gerät zur Stimulation des Bewegungsapparates und der Muskulatur, insbesondere im Bein- und Rückbereich bekannt, bei dem vorgesehen ist, dass an einem Gesteil zwei Trittflächen angeordnet sind, die mittels eines Antriebsmechanismus im Gegentakt oszillierend heb- und senkbar sind. Der Grundtonus der Muskulatur wird durch das Eigengewicht des auf den Trittflächen stehenden Patienten eingestellt, während die Muskelstimulation durch die oszillierende Trittftächenbewegung erfolgt.

Aus der US 2006/0229159 A1 ist ein Balance-Trainingsgerät bekannt, welches dazu dient, die Balancier-Fähigkeit eines Benutzers zu trainieren. Dieses Trainingsgerät weist eine Grundplatte auf, die von einem Motor angetrieben wird. Ein Drehwinkelsensor dient zur Erfassung des Drehwinkels der Grundplatte. Des weiteren ist eine Drehmomentmesseinrichtung vorgesehen, die dazu dient, das vom Benutzer auf die Grundplatte aufgebrachte Drehmoment zu messen. Die Ausgangssignale des Drehwinkelsensors und der Drehmomentmesseinrichtung werden einer Auswerteeinrichtung zugeführt, deren Ausgangssignal zu einem Motor-Controller geleitet wird, der dann entsprechende Steuersignale für den Motor erzeugt Hierzu wird in der Auswerteeinrichtung entsprechend eines vorgegebenen kinetischen Modells, welches die mechanischen Charakteristiken der Grundplatte vorgibt, die entsprechende Kompensationsdrehbewegung der Grundplatte berechnet und den Motor entsprechend geregelt.

Es erfolgt also eine Drehbewegung der Platte als Reaktion auf die vom Benutzer ausgelöste Drehmomentbeaufschlagung, die Auswerteeinheit fungiert hier als Regeleinrichtung, welche das Drehmoment des Motors entsprechend einem kinetischen Modell regelt. In das kinetische Modell gehen als virtuelle Parameter das Trägheitsmoment der Grundplatte, deren Federkonstante und deren Dämpfung ein, so dass durch eine entsprechende Auswahl einer oder mehrerer der vorgenannten virtuellen Parameter unterschiedlich träge, federnde und/oder dämpfende Grundplatten simuliert und derart unterschiedliche Trainingsbedingungen und somit Balance-Anforderungen vorgegeben werden können. Das vom Benutzer vorgesehene Drehmoment wird dadurch gemäß dem vorgegebenen kinetischen Modell und den bei dieses eingehenden virtuellen Parametern durch den Motor kompensiert. Die bekannte Vorrichtung erlaubt somit lediglich durch die Simulation unterschiedlicher Grundplatten die Bereitstellung von unterschiedlichen Trainingsbedingungen, ist aber nicht in der Lage, Aufschlüsse über Parameter des Bewegungsapparates des Benutzers zu geben. Somit wird insbesondere nicht der zeitliche Zusammenhang der Verläufe der Messgrößen berücksichtigt.

Die DE 611 11 016 T2 beschreibt eine automatische Vorrichtung zur optimierten Muskelstimulierung wobei die Stimulierung für periodische Kontraktionen eines oder mehrerer Muskel eines Benutzers mit konstanter Frequenz sorgt. Hierzu sind ein oder mehrere Detektoren vorgesehen, die jeweils auf einen entsprechend zu stimulierenden Muskeln des Benutzers aufgebracht werden. Dann wird eine optimale Frequenz der periodischen Muskelkontraktion innerhalb eines Bereichs zwischen einer unteren Grenzfrequenz und einer oberen Grenzfrequenz aufgrund entsprechender periodischer mechanischer Stimulierung durch mechanische Mittel vorbestimmt, entsprechend welcher die Summe der Amplituden der Signale, die von den Detektoren von den entsprechend stimulierten Muskeln als Reaktion auf die Stimulierung geliefert werden, die größte ist, und die mechanischen Mittel erzeugen dann eine periodische Kontraktion der Muskeln mit der vorbestimmten optimalen Frequenz. Dieses Gerät sieht also vor, dass mittels Sensoren eine optimale Stimulierungsfrequenz der Muskeln bestimmt wird und dann die mechanischen Mitteln dementsprechend justiert werden.

Die WO 2006/070429 beschreibt ein Trainingsgerät, welches wiederum auf eine vorbestimmte, optimale Muskelkontraktionsfrequenz abgestimmt ist.

Es ist daher die Aufgabe der vorliegenden Erfindung, eine Vorrichtung und ein Verfahren der eingangs genannten Art derart weiterzubilden, dass zur Analyse des Bewegungsapparats des Benutzers eine korrelierte Auswertung der auf den Benutzer aufgeprägten Anregungsfunktion und der daraus resultierenden Antwortfunktion des Benutzers möglich ist.

Diese Aufgabe wird erfindungsgemäß dadurch gelöst, dass die Vorrichtung mindestens einen weiteren Sensor aufweist, durch den die Position und/oder der Bewegungszustand des die Anregungsfunktion der Vorrichtung auf den Benutzer aufprägenden Grundelements erfassbar ist und dessen oder deren Sensorsignale der Auswerteeinrichtung zuführbar ist, und dass durch die Auswerteeinrichtung ein Vergleich der durch das Grundelement auf den Benutzer aufgeprägten Anregungsfunktion und der Antwortfunktion des Benutzers durchführbar ist, indem die Auswerteeinrichtung die Amplitude und/oder die Phasenlage dieser beiden Funktionen zur Bestimmung von Parametern des Bewegungsapparates des Benutzers analysiert.

Das erfindungsgemäße Verfahren sieht vor, dass die Anregungsfunktion der Vorrichtung mittels mindestens eines Sensors erfasst und in einer Auswerteeinrichtung die von der Vorrichtung erzeugte auch auf den Benutzer einwirkende Anregungsfunktion mit der aus der auf den Benutzer aufgeprägten Anregungsfunktion resulderende Antwortfunktion des Benutzers verglichen wird, indem die Auswerteeinriehtung die Amplitude und/oder die Phasenlage dieser beiden Funktionen zur Bestimmung von Parametern des Bewegungsapparates des Benutzers analysiert.

Durch die erfindungsgemäßen Maßnahmen wird in vorteilhafter Art und Weise erreicht, dass die Anregungsfunktion, welche die erfindungsgemäße Vorrichtung auf ihren Benutzer ausübt, und dessen Antwortfunktion darauf zu einander in Beziehung gesetzt werden können. Dadurch kann eine Übertragungsfunktion, welche die vom Grundelement auf den Benutzer aufgeprägte Anregungsfunktion auf die Antwortfunktion des Benutzers abbildet, bestimmt werden. Aus dieser Obertragungsfunktion lassen sich dann Rückschlüsse über die Funktion - und insbesondere über Fehlfunktionen - des Bewegungsapparates eines bestimmten Benutzers ziehen und in einfacher Art und Weise viele Funktionen diagnostizieren. Ausgehend davon kann dann das Training des Bewegungsapparates des Benutzers verbessert werden.

Vorteilhafte Weiterbildungen der Erfindung sind Gegenstand der Unteransprüche.

Weitere Einzelheiten und Vorteile der Erfindung sind den Ausführungsbeispielen zu entnehmen, die im folgenden anhand der Figuren beschrieben werden.

Es zeigen:
- Figur 1:: ein erstes Ausführungsbeispiel einer Vorrichtung,
- Figur 2:: eine Draufsicht auf das erste Ausführungsbeispiel aus der Richtung II der Figur 1,
- Figur 3:: ein zweites Ausführungsbeispiel einer Vorrichtung,
- Figur 4:: ein drittes Ausführungsbeispiel einer Vorrichtung,
- Figur 5:: eine Draufsicht auf das dritte Ausführungsbeispiel aus der Richtung V der Figur 4,
- Figur 6:: ein viertes Ausführungsbeispiel einer Vorrichtung,
- Figur 7:: eine Draufsicht auf das vierte Ausführungsbeispiel aus Richtung VII der Figur 6,
- Figur 8:: eine schematische Darstellung einer Messanordnung,
- Figur 9:: ein fünftes Ausführungsbeispiel der Vorrichtung, und
- Figur 10:: ein sechstes Ausführungsbeispiel einer Vorrichtung.

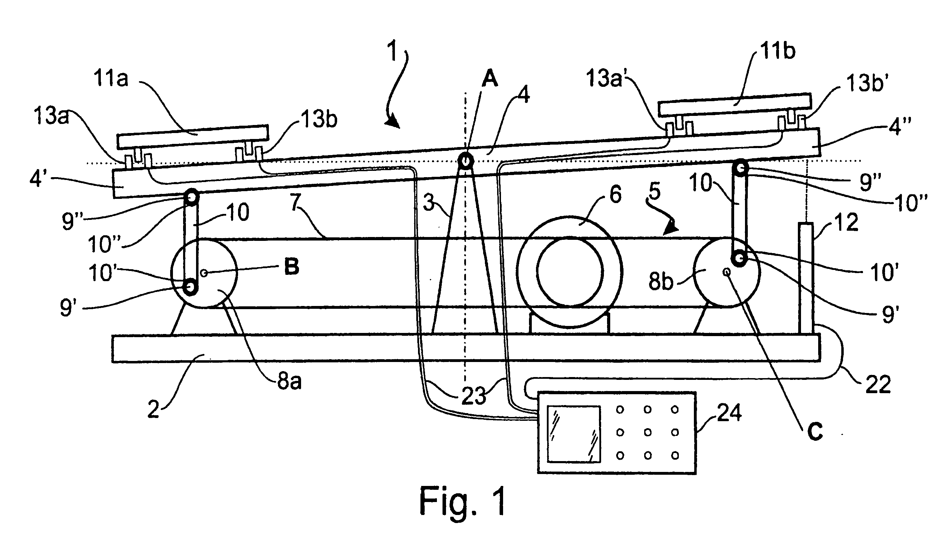

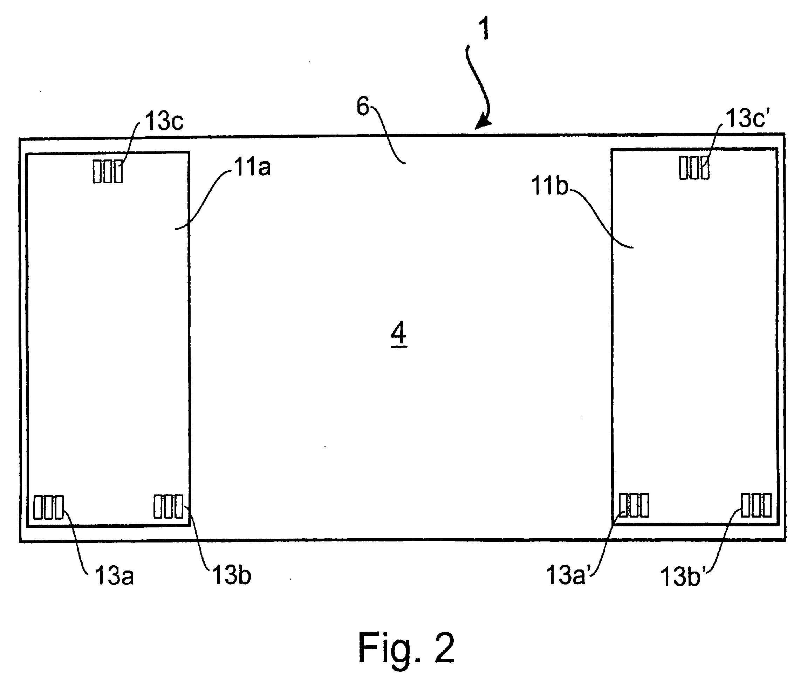

Die Figuren 1 und 2 zeigen nun ein erstes Ausführungsbeispiel einer allgemein mit 1 bezeichneten Vorrichtung für ein Training und/oder eine Analyse des Bewegungsapparats eines Lebewesens, insbesondere eines Menschen. Die Vorrichtung 1 besitzt einen Sockel 2 mit einem Ausleger 3, an dem ein um eine Achse A drehbar gelagertes Grundelement 4 angeordnet ist, welches hier als Grundplatte ausgeführt ist. Das im gezeigten Ausführungsbeispiel als Wippe fungierende Grundelement 4 wird von einer Antriebseinrichtung 5 angetrieben, welche dazu dient, das Grundelement 4 in Schwingungen zu versetzen. Im hier gezeigten Fall weist die Antriebseinrichtung 5 einen Elektromotor 6 auf, welcher über einen Riemen 7 zwei um Achsen B und C drehbar gelagerte Antriebsscheiben 8a und 8b antreibt. An einem ersten Anlenkpunkt 9' ist jeweils ein erstes Ende 10' eines Pleuels 10 angelenkt. Ein zweites Ende 10" eines jeden Pleuels 10 greift über einen weiteren Anlenkpunkt 9" an der Grundplatte 4 an, so dass durch eine Drehbewegung der Antriebsscheiben 8a, 8b die Grundplatte 4 in sinusförmige Schwingungen versetzt wird.

Dem Fachmann ist aus der obigen Beschreibung klar ersichtlich, dass die hier beschriebene Lagerung des Grundelements 4 sowie die Ausbildung der Antriebseinrichtung 5 nur exemplarischen Charakter besitzen. So ist es zum Beispiel auch möglich, dass das Grundelement 4 über entsprechende Führungen (nicht gezeigt) auf- und abbeweglich gelagert ist. Auch ist es nicht erforderlich, dass das Grundelement 4 an zwei Stellen angetrieben wird. Ein Antrieb des Grundelements 4 an nur einer Stelle oder an mehr als zwei Stellen ist ebenfalls möglich, genauso wie ein Antrieb desselben über mehr als eine Antriebseinrichtung. Ebenso wenig ist es zwingend, dass das Grundelement 4 mit einer periodischen Anregung beaufschlagt wird. Auch eine aperiodische Beaufschlagung des Grundelements 4, insbesondere eine stochastische oder impulsförmige Beaufschlagung, ist möglich. Ebenso ist es denkbar, dass das Grundelement 4 anstelle von sinusförmigen Schwingungen oder zusätzlich dazu mit Vibrationen beaufschlagt wird. Vorzugsweise wird hierbei eine Frequenz verwendet, die im Bereich von 0,1 bis 15 Hz bzw. 10 bis 50 Hz liegt. Eine Anregung im erstgenannten Frequenzbereich besitzt den Vorteil, dass hierdurch der Regelkreis des postularen Systems gezielt untersucht werden kann. Der zweitgenannte Bereich erlaubt eine effiziente Analyse der Steifigkeit und Dämpfung des Bewegungsapparates. Dem Fachmann ist klar ersichtlich, welche Modifikationen er an dem beschriebenen Aufbau vorzunehmen hat, um bei der beschriebenen Vorrichtung 1 eine oder mehrere der vorstehend beschriebenen Möglichkeiten vorzusehen. Im folgenden soll jedoch der Einfachheit halber weiterhin von dem in Figur 1 gezeigten und vorstehend beschriebenen Aufbau ausgegangen werden, da dies die Allgemeingültigkeit der nachfolgenden Ausführungen nicht beeinträchtigt.

Die Antriebsbewegung des Elektromotors 6 sowie deren Übertragung über die Antriebsscheiben 8a, 8b und die Pleuel 10 auf das Grundelement 4 bewirkt, dass diese eine sinusförmige Bewegung durchführt, wobei einander gegenüberliegende Enden 4' und 4" des Grundelements 4 eine Phasenverschiebung von 180 **°** aufweisen. Der Bewegungsapparat eines auf dem Grundelement 4 stehenden Benutzers wird somit mit einer sinusförmigen Anregung beaufschlagt, woraufhin dieser nun versucht, die Anregungsbewegungen dem Grundelement 4 durch entsprechende Reaktionen seines Bewegungsapparates zu kompensieren. Vorzugsweise ist vorgesehen, dass diese sinusförmige Anregungsfunktion eine Frequenz im Bereich von 5 bis 50 Hz besitzt.

Auf dem Sockel 2 der Vorrichtung 1 ist ein Abstandssensor 12 angeordnet, durch den die Auslenkung des Grundelements 4 erfassbar ist. Sein Sensorsignal wird über eine Signalleitung 22 zu einer Auswerteeinrichtung 24 geführt und dort entsprechend verarbeitet. Der Abstandssensor 12 erlaubt eine kontinuierliche, quasi-kontinuierliche oder zu diskreten Zeitpunkten stattfindende Erfassung der Lage des Grundelements 4, wobei aus dem zeitlichen Verlauf der Auslenkungsamplituden des Grundelements 4 die Auswerteeinrichtung deren Frequenz und/oder Amplitude, insbesondere die zur aktuellen Auslenkung des Grundelements korrelierte Momentanamplitude, sowie daraus ableitbare Bewegungsparameter des Grundelements 4 ermitteln werden können. Bevorzugt wird hierbei, dass die Amplitude und/oder die Frequenz der Auslenkung des Grundelements 4 in seiner Wirkrichtung, im hier gezeigten Fall also in der horizontalen Richtung, erfasst bzw. ermittelt werden.

Anstelle des Abstandssensors 12 kann auch ein anderer Sensortyp, zum Beispiel ein Kraft-, Druck-, Weg-, Geschwindigkeits- oder Beschleunigungssensor eingesetzt werden, solange gewährleistet ist, dass durch den entsprechenden Sensor die Anregungsbewegung des Grundelements 4 der Vorrichtung 1 in seiner Wirkrichtung erfassbar ist.

Wie aus den Figuren 1 und 2 ersichtlich, weist das Grundelement 4 zwei Trittplattformen 11a, 11 b auf, welche dazu dienen, eine definierte Position des Benutzers auf dem als Grundplatte ausgebildeten Grundelement 4 festzulegen. Selbstverständlich sind diese Trittplattformen 11a, 11b nicht zwingend erforderlich. Es ist durchaus möglich, dass der Benutzer der Vorrichtung 1 direkt auf der Grundplatte steht.

Um nun zur Durchführung eines Trainings und/oder einer Analyse eines Bewegungsapparates des Benutzers dessen Antwort auf die durch die Vorrichtung 1 aufgebrachte Anregung ermitteln zu können, ist vorgesehen, dass die Vorrichtung 1 einen oder mehrere entsprechend ausgebildete Sensoren aufweist, mit denen die Antwort des Bewegungsapparats des Benutzers auf die Anregung erfassbar ist. Im hier beschriebenen Fall, bei dem auf der Grundplatte Trittplattformen 11a, 11 b angeordnet sind, ist vorgesehen, dass - wie am besten aus Figur 2 ersichtlich ist - im Bereich jeder Trittplattform 11a, 11 b drei Sensoren 13a-13c bzw. 13a'-13c' angeordnet sind, welche die vom Benutzer auf die Trittplattformen 11 a, 11 b und somit auf das Grundelement 4 eingeleiteten Druckkräfte erfassen und deren Sensorsignale über einen Sensorleitung 23 zu der Auswerteeinrichtung 24 geleitet werden. Durch eine kontinuierliche, quasi-kontinuierliche oder zu diskreten Zeitpunkten stattfindende Erfassung und/oder Auswertung der Sensorsignale der Sensoren 13a-13c, 13a'-13c' ist es somit möglich, die vom Benutzer auf das Grundelement 4 ausgeübten Druckkräfte in ihrem zeitlichen Verlauf zu erfassen sowie die daraus ableitbaren physikalischen Größen zu ermitteln. Dadurch ist es möglich, den zeitlichen Verlauf der Antwortfunktion des Benutzers auf die von der Vorrichtung 1 auf ihn ausgeübte Anregungsfunktion zu erfassen.

Die drei Sensoren 13a-13c, 13a'-13c' sind hierbei vorzugsweise nicht-kollinear angeordnet, so dass hierdurch nicht nur die vom Benutzer auf die jeweilige Trittplattform 11a, 11b aufgebrachten Druckkräfte erfassbar ist, sondern dass aus der räumlichen Beziehung der drei Sensoren 13a-13c; 13a'-13c' der Schwerpunkt der Kraftverteilung und somit der Gesamt-Krafteinleitpunkt ermittelbar ist. Dies erlaubt die genaue Erfassung der Position des Fußes des Benutzers auf der ersten und der zweiten Trittplattform 11a, 11 b, woraus wieder die genaue Amplitude der den Fuß beaufschlagende Anregung ermittelbar ist. Es ist aber auch möglich, weniger oder mehr Sensoren zu verwenden oder nur einen einzigen Sensor pro Trittplattform 11a, 11 b zu verwenden, der aber dann vorzugsweise auflösend ausgeführt ist. Die Verwendung eines oder mehrerer ortsauflösender Sensoren auf der oder den Trittplattformen 11a, 11b und/oder auf dem Grundelement 4 erlaubt in vorteilhafter Art und Weise eine örtlich aufgelöste Erfassung der vom Benutzer hervorgerufene Druck- oder Kraftverteilung. Wenn auf die Trittplattformen 11a, 11 b verzichtet werden soll, so kann dann der entsprechende Bereich des Grundelements 4 mit zur Erfassung der Krafteinleitung geeigneten Sensoren, insbesondere der vorstehend beschriebenen Sensoren, versehen sein.

Ein Vergleich dieser beiden Funktionen erlaubt es nun, die mit der Vorrichtung 1 bewirkte Anregung einerseits und die Reaktion des Benutzers andererseits zueinander in Beziehung zu setzen und durch die Auswertung insbesondere der Amplituden, der Kurvenformen, der Frequenzkomponenten der Kurvenformen und/oder der Phasenlage der beiden Funktionen auf Charakteristika und/oder eine etwaige Fehlfunktion des Bewegungsapparates zu schließen.

Beim ersten Ausführungsbeispiel ist die von dem Grundelement 4 auf den Benutzer aufgeprägte Anregungsfunktion eine sinusförmige Funktion, wobei die maximale Auslenkung des in der Figur 1 linken Endes 4' des Grundelements 4 bei einer Phasenlage von 90 ° bzw. 270 ° und die maximale Auslenkung des rechten Endes 4" des Grundelements 4 bei einer Phasenlage von 270 ° bzw. 90 ° stattfindet. Zum Zeitpunkt ihrer maximalen Auslenkung besitzt das Grundelement 4 aber die geringste Geschwindigkeit, welche entsprechend der sinusförmigen Anregung beim Nulldurchgang der Grundplatte 4, also bei einer Phasenlage von 0 ° bzw. 180°, am größten ist.

Betrachtet man nun den Bewegungsapparat des Benutzers als ein wegerregtes Feder-Masse-Dämpfungssystem, so würde bei einem reinen Feder-Masse-System die Phasenverschiebung zwischen der maximalen Auslenkung der Grundplatte 4 und dem Maximalwert der vom Benutzer auf das Grundelement 4 aufgebrachten Kraft 0°, bei einem rein dämpfenden System, bei dem die resultierende Kraft von der auf den Benutzer aufgebrachten Geschwindigkeit der Grundplatte 4 abhängt, hingegen 90 ° betragen. Analysiert man nun die Antwortfunktion des Benutzers auf die von der Vorrichtung 1 vorgegebene Anregungsfunktion der Vorrichtung 1 hinsichtlich der Amplitude und/oder der Phasenlage dieser beiden Funktionen, so können in einer dem Fachmann bekannten Art und Weise kinematische Kenngrößen des Bewegungsapparates wie Steifigkeitskoeffizient oder Dämpfungskoeffizient insbesondere im Rahmen des vorgenannten Feder-Masse-Dämpfungs-Systems bestimmt werden.

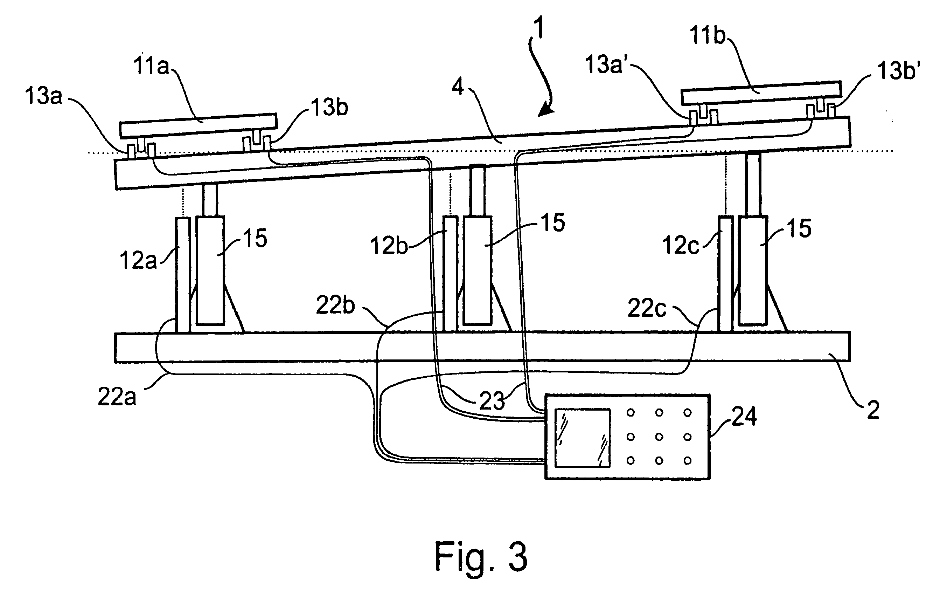

Die Figur 3 zeigt nun ein zweites Ausführungsbeispiel einer Vorrichtung 1, wobei einander entsprechende Teile mit den entsprechenden Bezugszeichen bezeichnet sind und nicht mehr näher beschrieben werden. Der wesentliche Unterschied zwischen dem ersten und dem zweiten Ausführungsbeispiel besteht in der Art und Weise, wie das Grundelement 4 in Bewegung versetzt wird, also in der Art und Weise der Erzeugung der Anregungsfunktion. Anstelle des Antriebs des Grundelements 4 über Pleuel 10 ist hier vorgesehen, dass das Grundelement 4 auf drei Linearaktoren 15 angeordnet ist, welche entsprechende Auf- und Abbewegungen durchführen und somit eine Bewegung des Grundelements 4 hervorrufen. Es bedarf keiner weiteren Ertäuterung, dass es auch möglich ist, anstelle der drei Linearaktoren 15 nur einen oder zwei Linearaktoren oder mehr als drei Linearaktoren zu verwenden. Eine derartige Ausgestaltung der Antriebseinrichtung 5 der Vorrichtung 1 ist insbesondere für eine aperiodische Anregungsfunktion geeignet, da hierdurch zum Beispiel Stöße, Vibrationen, Schwenk-, Kipp oder Drehbewegungen und/oder eine Kombination der vorgenannten Anregungen erzielbar sind. Die Vorrichtung 1 weist hier drei Abstandssensoren 12a-12c auf, mit denen die Lage des Grundelements 4 erfassbar ist.

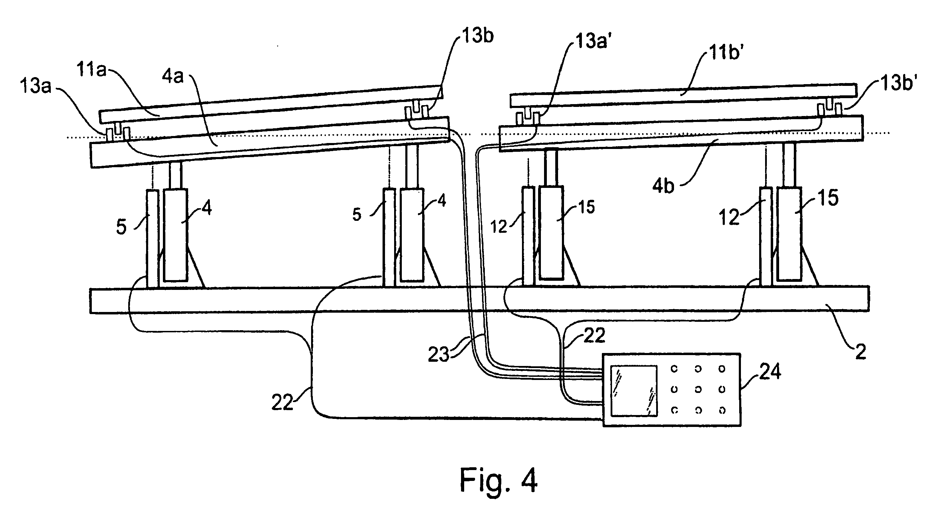

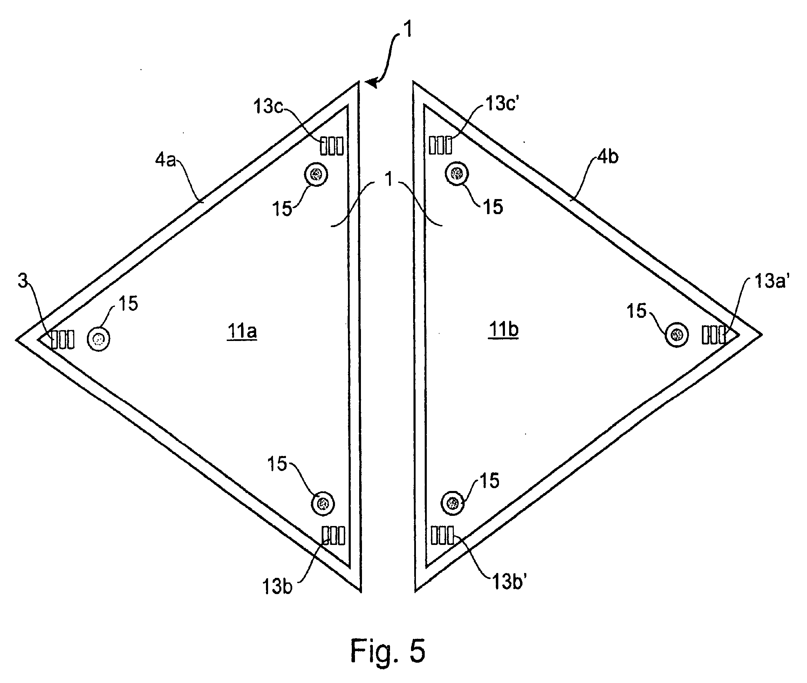

In den Figuren 4 und 5 ist ein drittes Ausführungsbeispiel einer Vorrichtung 1 dargestellt, wobei wiederum entsprechende Bauteile mit den gleichen Bezugszeichen versehen und nicht mehr näher beschrieben werden.

Der wesentliche Unterschied zu den beiden vorgenannten Ausführungsbeispielen besteht darin, dass das Grundelement 4 zweiteilig ausgeführt ist, dass also hier zwei Teil-Grundelemente 4a und 4b vorhanden sind, die zusammenwirkend die Funktion des Grundelements 4 der ersten beiden Ausführungsbeispiele realisieren. Der Vorteil einer derartigen Konstruktion ist, dass hier die beiden Teil-Grundelemente 4a und 4b unabhängig voneinander bewegt werden können. Die beschriebene Vorrichtung 1 erlaubt es somit in vorteilhafter Art und Weise, den Bewegungsapparat des Benutzers nur einseitig oder seitenspezifisch unterschiedlich stark anzuregen, indem zum Beispiel ein Teil-Grundelement 4a nicht und das zweite Teil-Grundelement 4b wie vorstehend in Schwingungen versetzt wird, oder dass die Amplitude und/oder die Frequenz der Schwingungen der beiden Teil-Grundelemente 4a, 4b unterschiedlich ausgeprägt sind. Insbesondere in diesem Fall ist es dann vorteilhaft, dass die dem jeweiligen Grund-Teilelementen 4a, 4b zugeordneten Sensoren getrennt ausgewertet werden.

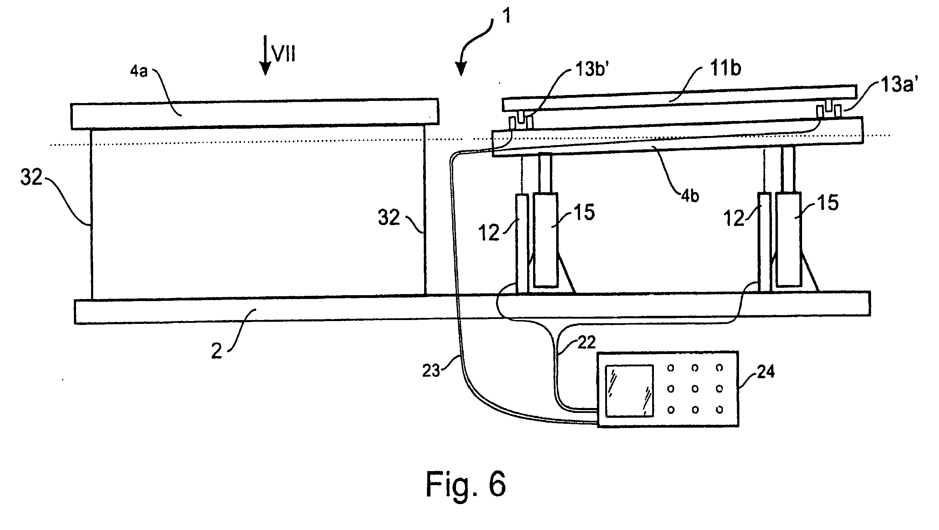

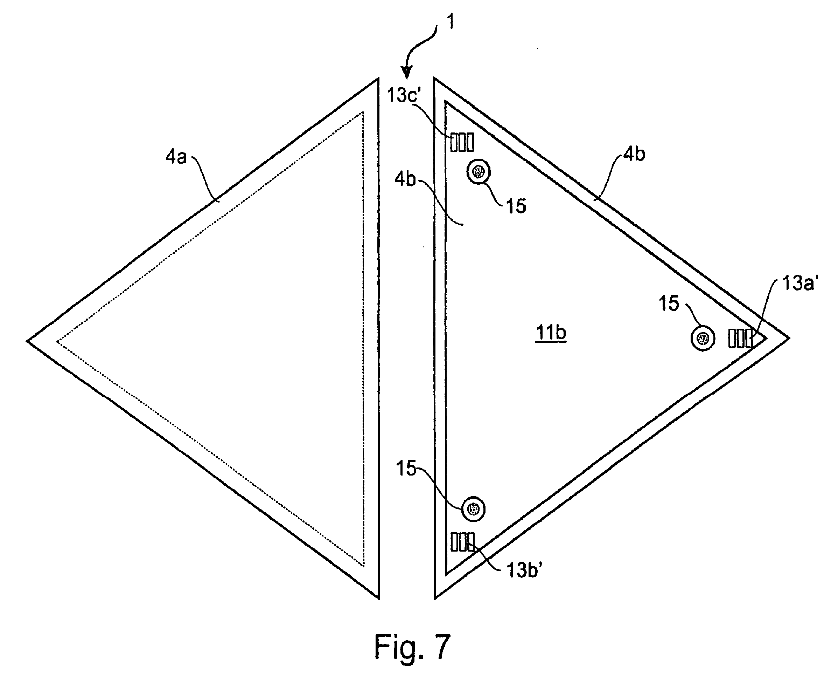

In den Figuren 6 und 7 ist ein weiteres Ausführungsbeispiel einer Vorrichtung 1 dargestellt, wobei wiederum entsprechende Teile mit den gleichen Bezugszeichen versehen und nicht mehr näher beschrieben werden. Die Vorrichtung 1 weist wiederum zwei Teil-Grundelemente 4a, 4b auf, wobei aber nur das in Figur 5 rechte Teil-Grundelement 4b in Schwingung versetzbar ist, während das in Figur 5 linke Teil-Grundelement 4a starr ausgebildet ist, indem das erste Teil-Grundelement 4a mit dem Sockel 2 über Trägerelemente 22 fest verbunden ist. Eine derartige Vorrichtung erlaubt in vorteilhafter Art und Weise eine gezielt einseitige Anregung des Bewegungsapparates des Benutzers.

Von besonderem Vorteil ist, wenn der Auswerteeinrichtung der Vorrichtung das Signal mindestens eines weiteren Sensors zuführbar ist, durch den oder die die Übertragung der Anregungsfunktion von den durch das Grundelement 4, 4a, 4b beaufschlagbaren Extremitäten des Benutzers auf einen weiteren Bereich des Bewegungsapparates erfassbar ist. Es wäre zum Beispiel möglich, Positions-, Weg-, Geschwindigkeits-, oder Beschleunigungssensoren an weiteren Körperteilen des Benutzers, insbesondere am Kopf, anzubringen und die Bewegung des Körperteils, insbesondere der des Kopfes, zu erfassen. Eine möglichst geringe Bewegung des Kopfes weist zum Beispiel auf eine große Elastizität der Wirbelsäule und der Rückenmuskulatur des Benutzers hin, eine starke Bewegung des Kopfes hingegen lässt auf eine geringe Elastizität, die zum Beispiel durch eine Versteifung der Rückenmuskulatur verursacht wird, schließen.

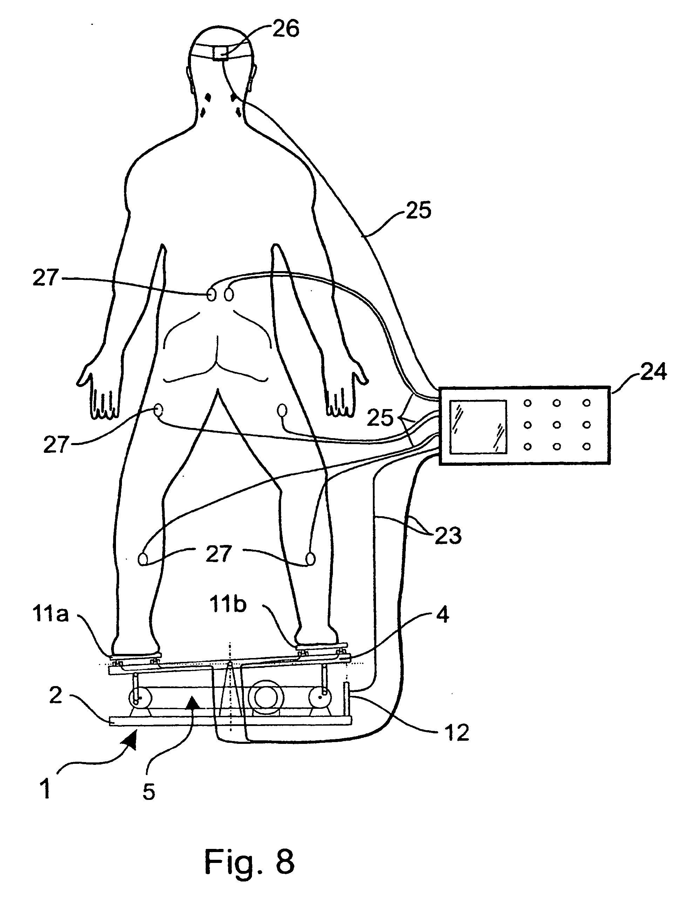

In Figur 8 ist nun eine typische Messanordnung dargestellt. Diese Figur zeigt einen auf der Vorrichtung 1 stehenden Benutzer, an dessen Kopf ein Beschleunigungssensor 26 angebracht ist, der über eine Signalleitung 25 mit der Auswerteeinrichtung 24 verbunden ist. Am Rumpf, an den Oberschenkeln und an den Unterschenkeln des Benutzers sind Elektroden 27 angeordnet, die zur Erfassung von elektrischen Strömen und Spannungen am Körper des Benutzers dienen, beispielsweise in Form von EMG (Elektromyogramm)-Sensoren, mit deren Hilfe die zeitlich korrelierte Aktivierung einzelner Muskelgruppen bzw. unterschiedlicher motorischer Einheiten innerhalb dieser Muskelgruppen erfasst werden kann. Eine derartige Erfassung ist insbesondere bei einer seitenalternierenden Anregung von Interesse, da hiermit die Erfassung der Aktivierung von Muskelgruppen im Seitenunterschied (z. B. an den Beinen, Rücken und Nacken) gezielt analysiert werden kann, wie sie zum Beispiel bei seitlichen Asymmetrien, wie sie of bei Rückenschmerzpatienten auftreten, gegeben sind.

Von weiterem Vorteil ist die Ergänzung der Messeinrichtung durch Sensoren zur Erfassung der Körperhaltung des Benutzers auf der Vorrichtung 1, zum Beispiel durch Verwendung eines ortsauflösenden Kraft- und Drucksensors in optionaler Kombination mit einem Gelenkwinkelsensor, zum Beispiel einem Goniomenter zur Erfassung des Kniewinkels. Auf diese Weise kann durch die Vorrichtung 1 in einfacher Art und Weise ermittelt werden, ob die Kräfte über den Vorfuß oder die Fersen in den Körper eingeleitet werden, was zur Aktivierung unterschiedlicher Muskelgruppen (z. B. vorwiegend Unterschenkel/Wade oder Oberschenkel) führt. Dies kann durch die zusätzliche oder alternative Auswertung des Kniewinkels weiter verfeinert werden. Die vorstehend beschriebenen zusätzlichen sensorischen Informationen erhöhen zum einen den Informationsgehalt der Analyse, zum anderen kann auf deren Basis ein Training auf der Vorrichtung 1 wesentlich besser gesteuert und somit deutlich optimiert werden.

Bei den vier vorstehend beschriebenen Ausführungsbeispielen wurde davon ausgegangen, dass das Grundelement 4 als eine Grundplatte ausgebildet ist, auf denen der Benutzer der Vorrichtung 1 mit einem oder beiden Füßen steht. Die Vorrichtung 1 ist aber nicht darauf beschränkt, dass über die Füße in den Bewegungsapparat des Benutzers die von dem Grundelement 4 vermittelte Anregungsfunktion eingeleitet wird. Es ist zum Beispiel auch möglich, dass das Grundelement 4 als ein Reck, als ein Griff oder als eine Hantel ausgebildet ist, so dass dann die Einleitung der Anregungsfunktion in den Bewegungsapparat des Benutzers über eine oder beide Hände erfolgt. Auch eine Kombination zwischen einem als Grundplatte ausgebildeten ersten Teil-Grundelement 4a und einem als Griff, etc. ausgebildeten zweiten Teil-Grundelement 4b ist möglich.

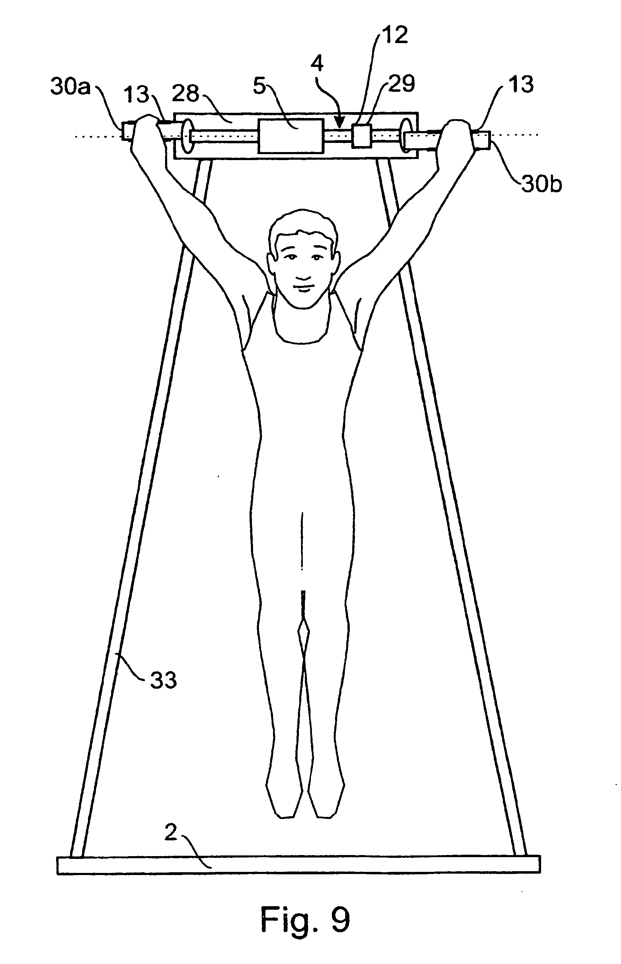

In Figur 9 ist nun eine rechteckig ausgebildete Vorrichtung 1 dargestellt. Auf dem Sockel 2 ist über Halter 33 ein reckstangenförmiges ausgebildetes Grundelement 4 angeordnet, an dessen Griffen 30a, 30b der Benutzer hängt. Eine Antriebseinrichtung 5 bewirkt die Beaufschlagung der Griffe 30a, 30b mit der entsprechenden Anregungsfunktion, wobei in den Hantelgriffen 30a, 30b vorzugsweise ein integrierter Kraft-/Drucksensor 13 angeordnet ist, dessen Sensorsignal auf in der vorstehend beschriebenen Weise zu der Auswerteeinrichtung 24 geleitet und dort wie erläutert ausgewertet wird. Die Anregungsfunktion der beiden Griffe 30a, 30b wird über einen Sensor 13 ermittelt, der hier als Inkrementalgeber 29 ausgebildet ist. Das Sensorsignal des Sensors 13 wird wiederum zu der Auswerteeinrichtung 24 geführt und dort wie beschrieben ausgewertet.

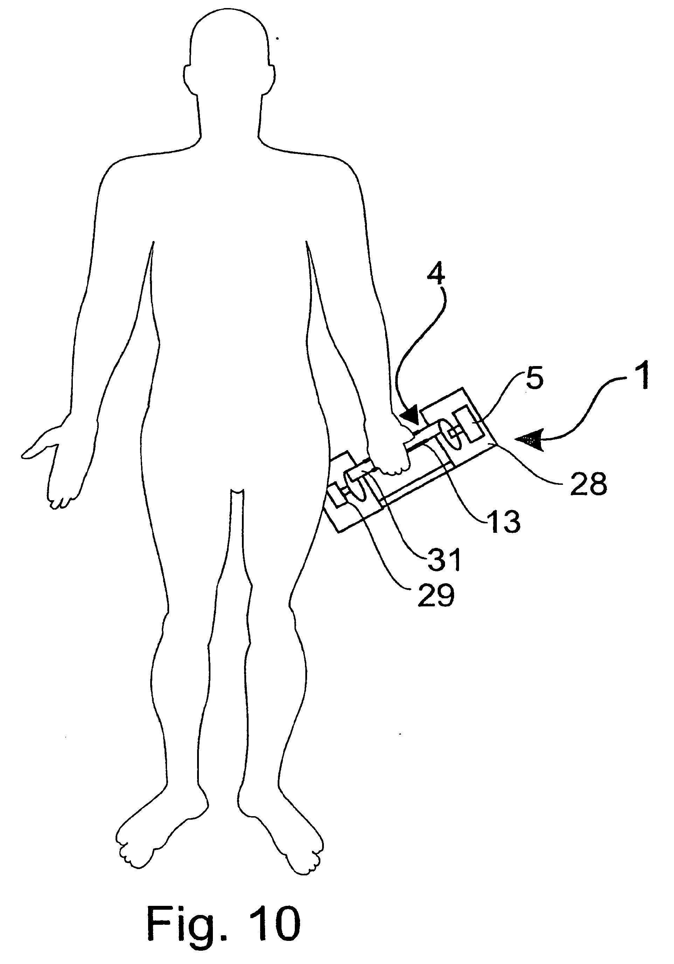

In Figur 10 ist ein weiteres Ausführungsbeispiel einer Vorrichtung 1 dargestellt, bei der das Grundelement 4 als Hantel ausgebildet ist. Eine derartige Hantel ist in dem europäischen Patent EP 0 929 2348 B1 sowie in der amerikanischen Patentanmeldung 734679 des Erfinders beschrieben. Die Antriebseinrichtung 5 sorgt für eine Beaufschlagung einer Hantelstange 31 mit der Anregungsfunktion, wobei die Hantelstange 31 wiederum einen Kraft-/Drucksensor 13 aufweist, dessen Ausgangssignal zu der Auswerteeinrichtung 24 geführt wird. Die Anregungsfunktion der Hantelstange 31 wird von einem Sensor 12 erfasst, dessen Ausgangssignal zu der Auswerteeinrichtung 24 geführt wird. Der Sensor 12 ist hier wiederum als Inkrementalgeber 29 ausgebildet. Die beiden vorstehend beschriebenen Ausführungsbeispiele gestatten es in besonders einfacher Art und Weise, die Antwortfunktion eine oder mehrer Arme und/oder des Oberkörperkörpers des Benutzers zu analysieren.

Die vorstehend beschriebene Vorrichtung 1 ist nicht nur zur Analyse des Bewegungsapparates eines Benutzers geeignet. Sie kann auch für Therapiezwecke verwendet werden, insbesondere für einen gezielten Aufbau von bestimmten Muskeln oder der Muskulatur allgemein, wobei durch einen Vergleich der Anregungsfunktion und der Antwortfunktion des Benutzers eine Optimierung der durch die Vorrichtung 1 hervorgerufenen Anregungsfunktion erzielbar ist.

Zusammenfassend ist festzustellen, dass durch die Vorrichtung 1 eine zum Training und/oder zur Analyse des Bewegungsapparates eines Benutzers besonders geeignete Vorrichtung geschaffen wurde, welche sich dadurch auszeichnet, dass durch einen Vergleich der von der Vorrichtung 1 bewirkten Anregungsfunktion auf den Bewegungsapparat und der Antwortfunktion zumindest eine Verbesserung, wenn nicht eine Optimierung, des Trainings und/oder der Analyse erzielbar ist.

## Patentansprüche

1. Vorrichtung für ein Training und/oder eine Analyse eines Bewegungsapparats eines Benutzers, wobei die Vorrichtung (1) mindestens ein Grundelement (4; 4a, 4b) , das durch eine Antriebseinrichtung (5) in periodische und/oder aperiodische Bewegungen zur Erzeugung einer auf den Benutzer einwirkenden Anregungsfunktion versetzbar ist, und mindestens einen Sensor (13: 13a-13c, 13a'-13c') zur Ermittlung einer Antwortfunktion eines Benutzers der Vorrichtung (1) auf die durch das Grundelement (4) auf ihn aufgeprägte Anregungsfunktion, dessen oder deren Sensorsignale einer Auswerteeinrichtung (24) der Vorrichtung (1) zugeführt sind, aufweist, **dadurch gekennzeichnet, dass** die Vorrichtung (1) mindestens einen weiteren Sensor (12; 12a-12c) aufweist, durch den die Position und/oder der Bewegungszustand des die Anregungsfunktion der Vorrichtung auf den Benutzer aufprägenden Grundelements (4; 4a, 4b) erfassbar ist und dessen oder deren Sensorsignale der Auswerteeinrichtung (24) zuführbar ist, und dass durch die Auswerteeinrichtung (24) ein Vergleich der durch das Grundelement (4; 4a, 4b) auf den Benutzer aufgeprägten Anregungsfunktion und der Antwortfunktion des Benutzers durchführbar ist, indem die Auswerteeinrichtung (24) die Amplitude und/oder die Phasenlage dieser beiden Funktionen zur Bestimmung von Parametern des Bewegungsapparates des Benutzers vergleicht.

2. Vorrichtung nach Anspruch 1, **dadurch gekennzeichnet, dass** der weitere Sensor (12; 12a-12c) als ein Abstandssensor ausgebildet ist, durch den die Lage des Grundelements (4) erfassbar ist.

3. Vorrichtung nach Anspruch 1, **dadurch gekennzeichnet, dass** der weitere Sensor (12; 12a-12c) als ein Kraft-, Druck-, Weg-, Geschwindigkeits- oder Beschleunigungssensor ausgebildet ist.

4. Vorrichtung nach einem der vorangehenden Ansprüche, **dadurch gekennzeichnet, dass** die Auswerteeinrichtung (24) das oder die Sensorsignale des oder der weiteren Sensoren (12; 12a-12c) kontinuierlich, quasikontinuierlich oder zu diskreten Zeitpunkten auswertet.

5. Vorrichtung nach einem der vorangehenden Ansprüche, **dadurch gekennzeichnet, dass** auf dem Grundelement (4; 4a, 4b) mindestens ein Sensor **(13a-13c;** 13a'-13c') angeordnet ist, durch den die vom Benutzer auf das Grundelement (4; 4a, 4b) aufgebrachte Kraft und/oder der Druck erfassbar ist.

6. Vorrichtung nach Anspruch 1 oder 5, **dadurch gekennzeichnet, dass** mindestens ein Sensor ortsauflösend ausgebildet ist.

7. Vorrichtung nach einem der vorangehenden Ansprüche, **dadurch gekennzeichnet, dass** das Grundelement (4) einteilig ausgebildet ist.

8. Vorrichtung nach einem der vorangehenden Ansprüche 1-6, **dadurch gekennzeichnet, dass** das Grundelement (4) mindestens zwei Teil-Grundelemente (4a, 4b) aufweist.

9. Vorrichtung nach einem der vorangehenden Ansprüche, **dadurch gekennzeichnet, dass** das Grundelement (4; 4a, 4b) als eine Grundplatte ausgebildet ist.

10. Vorrichtung nach einem der vorangehenden Ansprüche, **dadurch gekennzeichnet, dass** die Vorrichtung (1) mindestens eine Trittplattform (11a, 11 b) aufweist.

11. Vorrichtung nach Anspruch 10, **dadurch gekennzeichnet, dass** unter der oder unter mindestens einer der Trittplattformen (11a, 11b) mindestens ein Sensor (13a-13c; 13a'-13c') angeordnet ist.

12. Vorrichtung nach Anspruch 1, **dadurch gekennzeichnet, dass** das Grundelement (4) der Vorrichtung reckartig ausgebildet ist.

13. Vorrichtung nach Anspruch 11, **dadurch gekennzeichnet, dass** das reckartlg ausgebildete Grundelement (4) zwei Griffe (30a, 30b) aufweist, die von der Antriebseinrichtung (5) mit der Anregungsfunktion beaufschlagbar sind.

14. Vorrichtung nach Anspruch 1, **dadurch gekennzeichnet, dass** das Grundelement (4) der Vorrichtung (1) hantelartig ausgebildet ist.

15. Vorrichtung nach Anspruch 14, **dadurch gekennzeichnet, dass** das hantelartig ausgebildete Grundelement (4) eine Hantelstange (31) aufweist, die von der Antriebseinrichtung (5) mit der Anregungsfunktion beaufschlagbar ist.

16. Vorrichtung nach einem der vorangehenden Ansprüche, **dadurch kennzeichnet, dass** der Auswerteeinrichtung (24) der Vorrichtung (1) Sensorsignale mindestens eines weiteren, vorrichtungsexternen Sensors (26, 27) zuführbar sind.

17. Vorrichtung nach einem der vorangehenden Ansprüche **dadurch gekennzeichnet dass** die Vorrichtung mehrere Elektroden (27) aufweist, durch welche elektrische Ströme und/oder Spannungen am Körper des Benutzers erfassbar sind.

18. Vorrichtung nach Anspruch 16 oder 17 **dadurch gekennzeichnet, dass** durch den mindestens einan vorrichtungsexternen Sensor (26) und/oder die Elektroden (27) die Übertragung der Anregungsfunktion auf den Bewegungsapparat des Benutzers erfassbar ist.

19. Verfahren zum Training und/oder zur Analyse eines Bewegungsapparates eines Benutzers, bei dem durch eine Vorrichtung (1) zur Erzeugung einer Anregungsfunktion mechanische Reize auf den Benutzer aufgebracht werden, wobei die Antwortfunktion des Benutzers auf die von der Vorrichtung (1) erzeugte Anregungsfunktion erfasst wird, **dadurch gekennzeichnet, dass** die Anregungsfunktion der Vorrichtung (1) mittels mindestens eines Sensors (12; 12a-12c) erfasst und in einer Auswerteeinrichtung (24) die von der Vorrichtung (1) erzeugte auch auf den Benutzer einwirkende Anregungsfunktion mit der aus der auf den Benutzer aufgeprägten Anregungsfunktion resultierende Antwortfunktion des Benutzers verglichen wird, indem die Auswerteeinrichtung (24) die Amplitude und/oder die Phasenlage dieser beiden Funktionen zur Bestimmung von Parametern des Bewegungsapparates des Benutzers analysiert.

## Claims

1. Apparatus for exercise and/or analysis of the locomotor system of a user, the apparatus (1) having at least one basic element (4; 4a, 4b), which can be put into periodic and/or aperiodic movements by a driving device (5) for generating an excitation function acting upon the user, and having at least one sensor (13; 13a-13c, 13a'-13c') for detecting a response function of the user of the apparatus (1) to the excitation function applied to the basic element (4), the sensor signals of the sensor or the sensors are led to an evaluation device (24) of the apparatus (1), **characterized in that** the apparatus (1) has at least one further sensor (12; 12a-12c), by which the position and/or the state of movement of the basic element (4; 4a, 4b) excited by the excitation function of the apparatus (1) and acting upon the user can be captured and its or their sensor signals are leadable to the evaluation device (24), and that the evaluation device (24) is capable of performing a comparison of the excitation function applied to the user by the basic element (4; 4a, 4b) and the response function of the user **in that** the evaluation device (24) compares the amplitude and/or the phasing of these two functions for the determination of parameters of the locomotor system of the user.

2. Apparatus according to claim 1, **characterized in that** the further sensor (12; 12a-12c) is a distance sensor by which the position of the basic element (4) can be detected.

3. Apparatus according to claim 1, **characterized in that** the further sensor (12; 12a-12c) is a force sensor, a pressure sensor, a path sensor, a velocity sensor or an acceleration sensor.

4. Apparatus according to one of the previous claims, **characterized in that** the evaluation device (24) evaluates the sensor signal or the sensor signals of the further sensor or the further sensors (12; 12a-12c) continuously, quasi-continuously or at discrete points in time.

5. Apparatus according to one of the previous claims, **characterized in that** on the basic element (4; 4a, 4b) at least one sensor (13a-13c; 13a'-13c') is arranged, by which the force and/or the pressure exerted by the user on the basic element (4; 4a, 4b) can be detected.

6. Apparatus according to claim 1 or 5, **characterized in that** at least one sensor is position sensitive.

7. Apparatus according to one of the previous claims, **characterized in that** the basic element (4) is formed as one-piece.

8. Apparatus according to one of the previous claims 1-6, **characterized in that** the basic element (4) has got at least two basic element parts (4a, 4b).

9. Apparatus according to one of the previous claims, **characterized in that** the basic element (4; 4a, 4b) is provided as a ground plate.

10. Apparatus according to one of the previous claims, **characterized in that** the apparatus (1) has got at least one foot platform (11 a, 11 b).

11. Apparatus according to claim 10, **characterized in that** below the foot platform or below at least one of the foot platforms (11 a, 11 b) at least one sensor (13a-13c; 13a'-13c') is arranged.

12. Apparatus according to claim 1, **characterized in that** the basic element (4) of the apparatus is formed highbar-like.

13. Apparatus according to claim 12, **characterized in that** the highbar-like basic element (4) has got two grips (30a, 30b), which can be driven by the excitation function by the driving device (5).

14. Apparatus according to claim 1, **characterized in that** the basic element (4) of the apparatus (1) is formed dumbbell-like.

15. Apparatus according to claim 14, **characterized in that** the dumbbell-like basic element (4) has got a dumbbell bar (31), which can be driven by the excitation function by the driving device (5).

16. apparatus according to one of the previous claims, **characterized in that** sensor signals of at least one further, external sensor (26, 27) can be lead to the evaluation device (24).

17. Apparatus according to one of the previous claims, **characterized in that** the apparatus includes further electrodes (27), by which electric currents and/or electric tensions of the body of the user can be gathered.

18. Apparatus according to claim 16 or claim 17, **characterized in that** the transmission of the excitation function on the locomotor system of the user can be gathered by the at least one external sensor (26) and/or the electrodes (27).

19. Method for the training and/or analysis of the locomotor system of the user, where by means of an apparatus (1) for generating an excitation function mechanical excitations are impacted upon the user, and wherein the response function of the user to the excitation function generated by the apparatus (1) is gathered, **characterized in that** the excitation function of the apparatus (1) is gathered via at least one sensor (12; 12a-12c), and that in an evaluation device (24) the excitation function generated by the apparatus (1) and acting upon the user is compared with a response function of the user **in that** the evaluation device (24) compares the amplitude and/or the phasing of these two functions for the determination of parameters of the locomotor system of the user.

## Revendications

1. Système d'entrainement et/ou d'analyse de l'appareil moteur d'un utilisateur, ledit système (1) comportant au moins un élément de base (4) pouvant être mis en mouvement périodique et/ou apériodique par un dispositif d'entraînement (5), et au moins un capteur (13 ; 13a-13c, 13a'-13c') permettant de détecter une fonction de réponse d'un utilisateur du système (1) à une fonction d'excitation exercée par l'élément de base (4) sur celui-ci, et dont le ou les signaux de capteur sont adressables à un dispositif d'analyse (24) du système (1), **caractérisé en ce que** ledit système (1) comporte au moins un autre capteur (12 ; 12a-12c) permettant de saisir la position et/ou le mouvement de l'élément de base (4 ; 4a, 4b) exerçant la fonction d'excitation du système sur l'utilisateur, et dont le ou les signaux de capteur sont adressables au dispositif d'analyse (24), et **en ce qu'**une comparaison peut être effectuée par le dispositif d'analyse (24) entre la fonction d'excitation exercée par l'élément de base (4 ; 4a, 4b) sur l'utilisateur et la fonction de réponse de l'utilisateur, le dispositif d'analyse (24) comparant l'amplitude et/ou la position de phase de ces deux fonctions pour déterminer des paramètres de l'appareil moteur de l'utilisateur.

2. Système selon la revendication 1, **caractérisé en ce que** l'autre capteur (12 ; 12a-12c) est réalisé comme un capteur de distance permettant de saisir la position de l'élément de base (4).

3. Système selon la revendication 1, **caractérisé en ce que** l'autre capteur (12 ; 12a-12c) est réalisé comme un capteur de force, de pression, de course, de vitesse ou d'accélération.

4. Système selon l'une des revendications précédentes, **caractérisé en ce que** le dispositif d'analyse (24) analyse le ou les signaux du capteur ou des autres capteurs (12 ; 12a-12c) de manière continue, quasi-continue ou à des moments discrets.

5. Système selon l'une des revendications précédentes, **caractérisé en ce qu'**au moins un capteur (13a-13c ; 13a'-13c') est disposé sur l'élément de base (4 ; 4a, 4b), permettant de saisir la force et/ou la pression appliquée sur l'élément de base (4 ; 4a, 4b) par l'utilisateur.

6. Système selon la revendication 1 ou la revendication 5, **caractérisé en ce qu'**au moins un capteur est réalisé avec une résolution spatiale.

7. Système selon l'une des revendications précédentes, **caractérisé en ce que** l'élément de base (4) est réalisé en une seule pièce.

8. Système selon l'une des revendications 1 à 6, **caractérisé en ce que** l'élément de base (4) comprend au moins deux parties d'élément de base (4a, 4b).

9. Système selon l'une des revendications précédentes, **caractérisé en ce que** l'élément de base (4 ; 4a, 4b) est réalisé comme une plaque de base.

10. Système selon l'une des revendications précédentes, **caractérisé en ce que** le système (1) comporte au moins une plate-forme d'appui (11a, 11b).

11. Système selon la revendication 10, **caractérisé en ce qu'**au moins un capteur (13a-13c ; 13a'-13c') est disposé sous la plate-forme d'appui, ou sous au moins une des plates-formes d'appui (11a, 11 b).

12. Système selon la revendication 1, **caractérisé en ce que** l'élément de base (4) du système est réalisé en forme de barre fixe.

13. Système selon la revendication 11, **caractérisé en ce que** l'élément de base (4) réalisé en forme de barre fixe comporte deux poignées (30a, 30b) pouvant recevoir la fonction d'excitation du dispositif d'entraînement (5).

14. Système selon la revendication 1, **caractérisé en ce que** l'élément de base (4) du système (1) est réalisé en forme d'haltère.

15. Système selon la revendication 14, **caractérisé en ce que** l'élément de base (4) réalisé en forme d'haltère comporte une barre d'haltère (31) pouvant recevoir la fonction d'excitation du dispositif d'entraînement (5).

16. Système selon l'une des revendications précédentes, **caractérisé en ce que** des signaux de capteur d'au moins un autre capteur (26, 27) externe au système sont adressables au dispositif d'analyse (24) du système (1).

17. Système selon l'une des revendications précédentes, **caractérisé en ce que** le système comporte plusieurs électrodes (27) permettant de saisir des courants électriques et/ou des tensions sur le corps de l'utilisateur.

18. Système selon la revendication 16 ou 17, **caractérisé en ce que** la transmission de la fonction d'excitation vers l'appareil moteur de l'utilisateur peut être saisie par le ou les capteurs (26) externes au système et/ou par les électrodes (27).

19. Procédé d'entraînement et/ou d'analyse de l'appareil moteur d'un utilisateur, où des stimulations mécaniques sont exercées sur l'utilisateur par un système (1) pour la génération d'une fonction d'excitation, la fonction de réponse de l'utilisateur à la fonction d'excitation générée par le système (1) étant saisie, **caractérisé en ce que** la fonction d'excitation du système (1) est saisie par au moins un capteur (12 ; 12a-12c) et **en ce que** ladite fonction d'excitation est comparée à la fonction de réponse de l'utilisateur dans un dispositif d'analyse (24), le dispositif d'analyse (24) analysant l'amplitude et/ou la position de phase de ces deux fonctions pour déterminer des paramètres de l'appareil moteur de l'utilisateur.
